(19) Europäisches Patentamt
European Patent Office
Office européen des brevets

(11) Publication number : **0 429 187 B1**

# (12) EUROPEAN PATENT SPECIFICATION

(45) Date of publication of patent specification : **05.01.94 Bulletin 94/01**

(51) Int. Cl.$^5$ : **A61K 9/18,** A61K 31/565

(21) Application number : **90311738.0**

(22) Date of filing : **25.10.90**

## (54) Enhanced bioavailability adsorbates.

(30) Priority : **26.10.89 IE 3448/89**

(43) Date of publication of application : **29.05.91 Bulletin 91/22**

(45) Publication of the grant of the patent : **05.01.94 Bulletin 94/01**

(84) Designated Contracting States : **AT BE CH DE DK ES FR GB GR IT LI LU NL SE**

(56) References cited :
**EP-A- 0 163 178**
**EP-A- 0 232 155**
**EP-A- 0 274 176**
**GB-A- 2 153 677**

(73) Proprietor : **ELAN CORPORATION P.L.C.**
**Monksland Industrial Estate**
**Athlone County Westmeath (IE)**

(72) Inventor : **Bourke, Edward Anthony**
**704 Howth Road**
**Raheny, Dublin 5 (IE)**
Inventor : **Mulligan, Seamus**
**"Benown" , Glasson**
**Athlone, County Westmeath (IE)**

(74) Representative : **Ryan, Anne Mary et al**
**c/o Anne Ryan & Co. 60 Northumberland Road**
**Ballsbridge Dublin 4 (IE)**

EP 0 429 187 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to an adsorbate formulation for use in various drug delivery systems.

It is frequently desirable to enhance the bioavailability of an active substance from a pharmaceutical formulation in vivo. Various enhanced bioavailability pharmaceutical formulations are available which have a particular solubility profile, resulting in enhanced absorption of the active substance and, therefore, more effective medication.

The use of many active substances in therapy is complicated by problems of poor solubility and this is especially the case with the steroid group of drugs. DHEA and like steroids and salts thereof are insoluble and exhibit poor bioavailability due to poor absorption when administered orally, thus rendering them generally unsuitable for oral administration.

It is an object of the present invention to provide enhanced bioavailability formulations and, in particular, enhanced bioavailability steroid formulations resulting from improved absorption of the steroid component of the formulations.

Accordingly, the invention provides an enhanced bioavailability adsorbate formulation comprising an adsorbate of a mixture of one part by weight of a compound of the general formula (I)

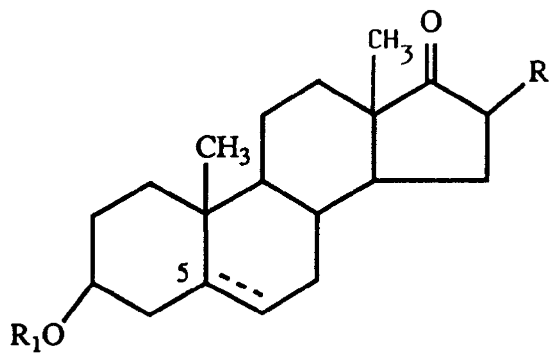

(I)

in which R is a hydrogen or bromine atom, and $R_1$ is a hydrogen atom, an $SO_2OM$ group wherein M is a hydrogen or sodium atom, a sulphatide group

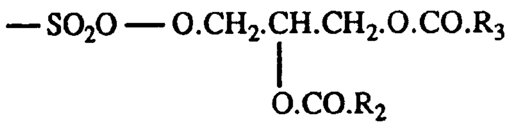

wherein each of $R_2$ and $R_3$, which may be the same or different, is a straight or branched chain alkyl radical of 1 to 14 carbon atoms,
a phosphatide group

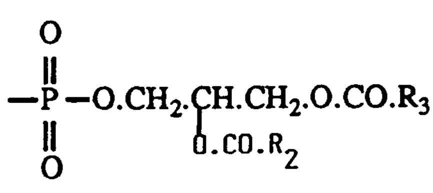

wherein each of $R_2$ and $R_3$, which may be the same or different, is a straight or branched chain alkyl radical of 1 to 14 carbon atoms,
or a glucuronide group

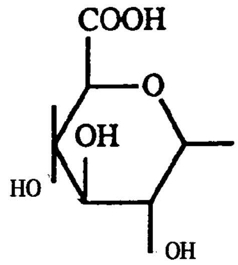

and wherein the broken line represents an optional double bond and the hydrogen atom at position 5 is present in the α- or β-configuration or a mixture of both configurations, and from 0.1 to 10 parts by weight of polyvinylpyrrolidone, adsorbed on a cross-linked polyvinylpyrrolidone in a ratio of 1 part by weight of said mixture to 0.20 to 20 parts by weight of cross-linked polyvinylpyrrolidone.

Preferred steroids covered by the general formula (I) are dehydroepiandrosterone (DHEA) and 16 - bromoepiandrosterone or hydrates, including polymorphs, enantiomers and isomers thereof or salts thereof, especially DHEA sulphate.

The formation of the DHEA adsorbate may result in the steroid being in an amorphous state to some extent, which can be verified by x-ray diffraction and, in addition, differential scanning calorimetry, which state may be retained in the enhanced bioavailability formulations hereinafter described.

In a preferred embodiment the polyvinylpyrrolidone is present in the adsorbate in an amount of 0.1 to 2 parts by weight relative to 1 part by weight of the steroid.

Furthermore in a preferred embodiment the formulation contains 1 part by weight of said mixture relative to 0.2 to 10 parts by weight of cross-linked polyvinylpyrrolidone.

A broad range of molecular weights of polyvinylpyrrolidones may be used (Mw 10,000 - 700,000) but a preferred polyvinylpyrrolidone is one where the average molecular weight is greater than 55,000, but is especially in the range 65,000 - 250,000.

The invention also provides a process for preparing an enhanced bioavailability formulation as defined above, which comprises dissolving the steroid and the polyvinylpyrrolidone in a common solvent in which both the steroid and the polyvinylpyrrolidone are soluble, mixing the solution thereby obtained with a given quantity of the cross-linked polyvinylpyrrolidone so as to permit adsorption of said steroid and said polyvinylpyrrolidone to said cross-linked polyvinylpyrrolidone and removing the solvent.

The solvent used may be any pharmaceutically suitable common solvent in which both the steroid and the polyvinylpyrrolidone are soluble.

The solvent is suitably selected from water, alcohols, ketones, halogenated aliphatic compounds, halogenated aromatic hydrocarbon compounds, aromatic hydrocarbon compounds and cyclic ethers or a mixture thereof.

Especially preferred solvents include water, methanol, ethanol, isopropyl alcohol, acetone, methylethyl ketone, methylisobutyl ketone, hexane, heptane, methylene chloride, dichloromethane, chloroform, carbon tetrachloride, toluene, xylene and tetrahydrofuran.

The polyvinylpyrrolidone is chosen to modify the solubility of the steroid in the presence of cross-linked polyvinylpyrrolidone and may also serve to maintain a level of amorphous drug in the polymer matrix structure of the absorbate during dissolution. Principally, the polyvinylpyrrolidone serves to aid the enhanced bioavailability of the dosage form according to the invention.

An especially preferred cross-linked polyvinylpyrrolidone is Crospovidone (sold under the Trade Marks POLYPLASDONE XL (GAF) and KOLLIDON CL (BASF)).

For manufacturing formulations according to the invention the absorbate may be blended with suitable excipients and used as a powder or dispersible granules, or, it may be granulated and blended with a polymer or mixture of polymers or mineral material which cause the formulation to disintegrate in the presence of water, and tabletted or encapsulated according to conventional methods. These formulations exhibit improved drug absorption and enhanced bioavailability. Suitable polymers for blending with adsorbates are inert polymers, which include water soluble polymers. Preferred polymeric and mineral materials which may be used include: natural starch (corn, potato, etc.) pregelatinised starch such as that sold under the Trade Mark AMIGEL, modified corn starch such as that sold under the Trade Mark STA R X 1500, sodium starch glycolate such as that sold under the Trade Marks PRIMOGEL and EXPLOTAB, sodium carboxymethylcellulose, carboxymethylcellulose, cellulose, methylcellulose, microcrystalline cellulose such as that sold under the Trade Mark AVICEL PH 101, ion exchange resins, cross-linked polyvinylpyrrolidone (Crospovidone) such as that sold under the Trade Marks POLYPLASDONE XL and KOLLIDON CL, clays such as aluminium magnesium silicate such as

that sold under the Trade Mark VEEGUM HV, bentonite, colloidal silicon dioxide such as that sold under the Trade Mark AEROSIL, alginic acid or salts thereof, and gums such as agar, guar, locust bean, karaya, pectin and tragacanth gums. Bentonite is native colloidal hydrated aluminium silicate freed from gritty particles and consisting mainly of montmorillonite ($Al_2O_3$ $4SiO_2$ $H_2O$). Bentonite swells in water to give a homogenous mass about 12 times the volume of the dry powder.

The adsorbates according to the present invention result in improved drug delivery relative to the micronised raw material, since the adsorbates in the formulations of the present invention yield an enhanced solubility complex leading to improved absorption and bioavailability of said active drug in vivo, and enhanced in vitro dissolution rates in excess of 1.1 times that of the micronised free steroid having a particle size less than 10 µm (D50).

In the accompanying drawings:

Fig. 1 represents biocurves denoting plasma levels (ng/ml) for DHEA base versus time (h) for a DHEA adsorbate in accordance with the invention (curve a) and standard micronised DHEA (curve b); and

Fig. 2 represents biocurves denoting plasma levels (µg/ml) for DHEA sulphate versus time (h) for a DHEA adsorbate in accordance with the invention (curve a) and standard micronised DHEA sulphate (curve b).

The invention will be further illustrated with reference to the following Examples.

EXAMPLE 1

An adsorbate is prepared having the following composition:

| | |
|---|---|
| DHEA | 57.143% w/w |
| Polyvinylpyrrolidone | 14.286% w/w |
| Crospovidone | 28.571% w/w |

The DHEA and polyvinylpyrrolidone are added to a suitable solvent such as acetone, dichloromethane or isopropanol and mixed until the solution is complete. Then the cross-linked polyvinylpyrrolidone (Crospovidone) is gradually added while mixing and the solvent evaporated. The resulting powder is reduced to obtain a finer particle size. X-ray diffraction and differential scanning calorimetry studies are performed on the powder and demonstrate that a proportion of the DHEA may be in an amorphous or altered crystalline state. The powder thus obtained is used to make several formulations as shown in the following Examples.

EXAMPLE 2

A powder formulation is prepared having the following composition:

| | |
|---|---|
| DHEA adsorbate (prepared as in Example 1) | 43.75% w/w |
| Sorbitol powder U.S.P. | 23.00% w/w |
| Aspartame (Aspartame is a Trade Mark) | 0.60% w/w |
| Lactose monohydrate U.S.P. | 32.65% w/w |

The above components are blended together and then a blend equivalent to 500 mg DHEA is packaged into suitable sachets.

EXAMPLE 3

A powder formulation is prepared having the following composition:

| | |
|---|---|
| DHEA adsorbate (prepared as in Example 1) | 21.88% w/w |
| Sorbitol powder U.S.P. | 11.50% w/w |
| Aspartame | 0.30% w/w |
| Lactose monohydrate U.S.P. | 66.32% w/w |

The above components are blended together and then a blend equivalent to 250 mg DHEA is packaged into suitable sachets.

EXAMPLE 4

A powder formulation is prepared having the following composition:

| | |
|---|---|
| DHEA adsorbate (prepared as in Example 1) | 65.624% w/w |
| Sorbitol powder U.S.P. | 33.576% w/w |
| Aspartame | 0.800% w/w |

The above components are blended together and then a blend equivalent to 750 mg DHEA is packaged into suitable sachets.

EXAMPLE 5

A capsule formulation is prepared having the following composition:

| | |
|---|---|
| DHEA adsorbate (prepared as in Example 1) | 87.5% w/w |
| Aerosil | 2.5% |
| Lactose monohydrate U.S.P. | 10.0% w/w |

The above components are blended together and then a blend equivalent to 400 mg DHEA is filled into suitable capsules.

EXAMPLE 6

An adsorbate formulation is prepared having the following composition:

| | |
|---|---|
| DHEA | 40.00% w/w |
| Polyvinylpyrrolidone | 20.00% w/w |
| Cross-linked polyvinylpyrrolidone | 40.00% w/w |

The DHEA and polyvinylpyrrolidone are added to a suitable solvent such as acetone, dichloromethane or isopropanol and mixed until the solution is complete. Then the cross-linked polyvinylpyrrolidone (POLYPLASDONE XL) is gradually added while mixing and the solvent evaporated.

EXAMPLE 7

A tablet formulation is prepared having the following composition:

| | |
|---|---|
| DHEA adsorbate (prepared as in Example 6) | 90.00% w/w |
| Cross-linked polyvinylpyrrolidone | 9.50% w/w |
| Magnesium stearate | 0.50% w/w |

The above components are blended together and then compressed to form 300 mg DHEA tablets.

EXAMPLE 8

An adsorbate is prepared having the following composition:

| | |
|---|---|
| DHEA sodium sulphate | 57.15% w/w |
| Polyvinylpyrrolidone | 14.28% w/w |
| POLYPLASDONE XL | 28.57% w/w |

The DHEA sodium sulphate and polyvinylpyrrolidone are added to a suitable solvent such as acetone, dichloromethane or isopropanol and mixed until the solution is complete. The POLYPLASIDONE XL is added gradually while mixing and the solvent is evaporated.

EXAMPLE 9

A powder formulation is prepared having the following composition:

| | |
|---|---|
| DHEA sodium sulphate adsorbate (prepared as in Example 8) | 45.87% w/w |
| Sorbitol powder U.S.P. | 23.00% w/w |
| Aspartame | 0.60% w/w |
| Lactose monohydrate U.S.P. | 30.53% w/w |

The above components are blended together and then a blend equivalent to 500 mg DHEA base is packaged into suitable sachets.

EXAMPLE 10

A capsule formulation is prepared having the following composition:

| | |
|---|---|
| DHEA sodium sulphate adsorbate (prepared as in Example 8) | 87.50% w/w |
| Aerosil | 2.50% w/w |
| Lactose Monohydrate U.S.P. | 10.00% w/w |

The above components are blended together and then a blend equivalent to 250 mg DHEA base is filled into suitable capsules.

Comparison of Enhanced Bioavailability DHEA Formulation with Micronised DHEA

In this study an enhanced bioavailability DHEA adsorbate powder of the type prepared in Example 2 was compared with standard micronised DHEA in capsule form. For the formulation in accordance with the invention (hereinafter referred to as 'test') the dose administered was 500 mg of DHEA as adsorbate in powder-sachet form. The standard micronised DHEA (hereinafter referred to as 'reference') was administered as 2 x 250 mg capsules. The micronised DHEA used had a particle size less than 10 μm (D50). In the study, the test substance was compared in a group of 9 healthy male human volunteers against the reference substance. The dosage regime was 500 mg every 8 hours over a 24 hour period. Plasma samples were taken in each case at intervals up to and including 24 hours post initial dosing and the samples were assayed for DHEA (base and sulphate) levels. DHEA sulphate levels were measured because a substantial portion of the DHEA is converted to the sulphate form in vivo. The levels were compared as shown in Table 1 and Figs. 1 and 2. For the reference substance, the area under the curve (AUC) value are arbitrarily denoted as 100%. The AUC value was then compared and assigned an absolute bioavailability value (%) using the 100% as baseline.

## TABLE 1

### Single Dose Data

Comparison of the relative bioavailability of the test substance with the reference using both DHEA base and DHEA sulphate.

| | REFERENCE (%) | TEST (%) |
|---|---|---|
| DHEA Base | *F = 100 | F = 127.25 |
| DHEA Sulphate | F = 100 | F = 129.39 |

*F = absolute bioavailability

Mean urinary recoveries for DHEA base for the reference and test substances are given in Table 2.

## TABLE 2

### Mean Urinary Recoveries for DHEA Base

| | | REFERENCE (mg/24 h.) | TEST (mg/24 h.) |
|---|---|---|---|
| DAY | 0 | 22.55 ± 8.50 | 24.66 ± 12.30 |
| | 1 | 460.67 ± 179.87 | 619.33 ± 220.54 |
| | 2 | 799.55 ± 191.90 | 800.78 ± 256.10 |
| | 3 | 745.88 ± 179.92 | 922.55 ± 238.52 |
| | 4 | 694.33 ± 162.07 | 827.66 ± 151.50 |
| | 5 | 800.22 ± 156.19 | 896.44 ± 173.27 |

**Claims**

**Claims for the following Contracting States : AT, BE, CH, LI, DE, DK, FR, GB, IT, LU, NL, SE**

1. An enhanced bioavailability adsorbate formulation comprising an adsorbate of a mixture of one part by weight of a compound of the general formula (I)

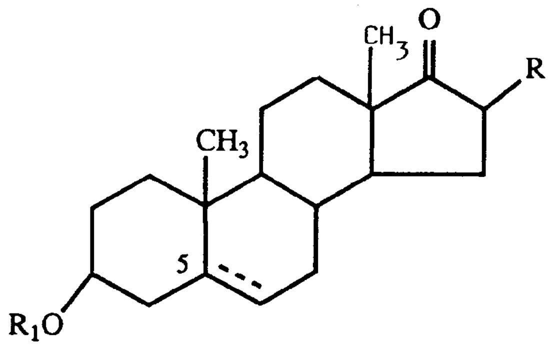

(I)

in which R is a hydrogen or bromine atom, and $R_1$ is a hydrogen atom, an $SO_2OM$ group wherein M is a hydrogen or sodium atom, a sulphatide group

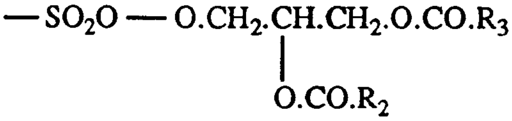

wherein each of $R_2$ and $R_3$, which may be the same or different, is a straight or branched chain alkyl radical of 1 to 14 carbon atoms,
a phosphatide group

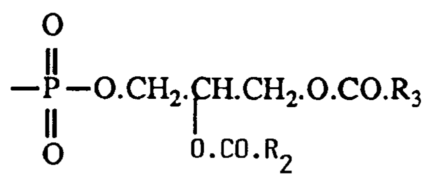

wherein each of $R_2$ and $R_3$, which may be the same or different, is a straight or branched chain alkyl radical of 1 to 14 carbon atoms,
or a glucuronide group

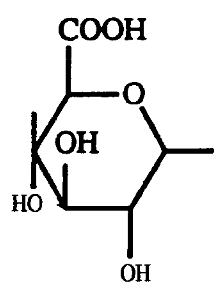

and wherein the broken line represents an optical double bond, and the hydrogen atom at position 5 is present in the α- or β-configuration or a mixture of both configurations, and from 0.1 to 10 parts by weight of polyvinylpyrrolidone, adsorbed on a cross-linked polyvinylpyrrolidone in a ratio of 1 part by weight of said mixture to 0.20 to 20 parts by weight of cross-linked polyvinylpyrrolidone.

2. A formulation according to Claim 1, characterised in that the compound is dehydroepiandrosterone or 16-bromoepiandrosterone, or a hydrate, polymorph, enantiomer or isomer thereof, or a salt thereof.

3. A formulation according to Claim 1 or 2, characterised in that the polyvinylpyrrolidone is present in the adsorbate in an amount of 0.1 - 2 parts by weight relative to 1 part by weight of the steroid.

4. A formulation according to any one of Claims 1 to 3, characterised in that it contains 1 part by weight of said mixture relative to 0.2 - 10 parts by weight of cross-linked polyvinylpyrrolidone.

5. A formulation according to any one of Claims 1 to 4, characterised in that the polyvinylpyrrolidone has an average molecular weight in the range 65,000 - 250,000.

6. A formulation according to any one of Claims 1 to 5, characterised in that the adsorbate is blended with a polymeric or mineral material which disintegrates in the presence of water.

7. A formulation according to Claim 6, characterised in that the material which disintegrates in the presence of water is selected from natural starch, pregelatinised starch, modified corn starch, sodium starch glycolate, sodium carboxymethylcellulose, carboxymethylcellulose, cellulose, methylcellulose, microcrystalline cellulose, ion-exchange resins, cross-linked polyvinylpyrrolidone, clays, alginic acid or salts thereof, and gums.

8. A process for preparing an enhanced bioavailability formulation according to any one of Claims 1 to 7, characterised in that it comprises the steps of dissolving the steroid and the polyvinylpyrrolidone in a common solvent in which both the steroid and the polyvinylpyrrolidone are soluble, mixing the solution thereby obtained with a given quantity of the cross-linked polyvinylpyrrolidone so as to permit adsorption of said steroid and said polyvinylpyrrolidone to said cross-linked polyvinylpyrrolidone and removing the solvent.

9. A formulation according to any one of Claims 1 to 7, characterised in that the in vitro solubility of the steroid in the absorbate is equal to or in excess of 1.1 times the solubility of the free steroid in micronised form.

10. A formulation according to any one of Claims 1 to 7 or 9, characterised in that it is in the form of a powder, granule, tablet, capsule or suspension.

**Claims for the following Contracting States : ES, GR**

1. A process for the preparation of an enhanced bioavailability adsorbate formulation comprising an adsorbate of a mixture of one part by weight of a compound of the general formula (I)

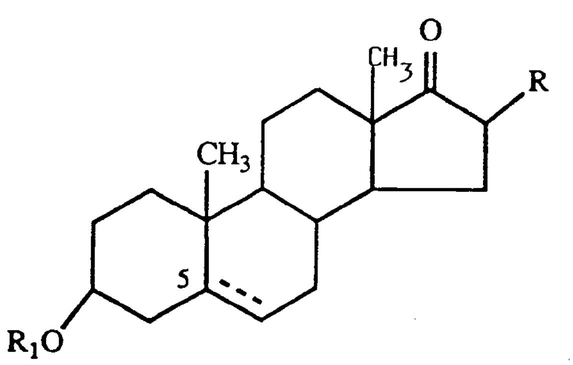

(I)

in which R is a hydrogen or bromine atom, and $R_1$ is a hydrogen atom, an $SO_2OM$ group wherein M is a hydrogen or sodium atom, a sulphatide group

$$-SO_2O-O.CH_2.\underset{\displaystyle |\atop \displaystyle O.CO.R_2}{CH}.CH_2.O.CO.R_3$$

wherein each of $R_2$ and $R_3$, which may be the same or different, is a straight or branched chain alkyl radical of 1 to 14 carbon atoms,
a phosphatide group

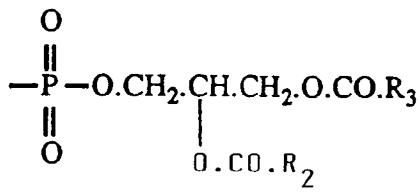

wherein each of $R_2$ and $R_3$, which may be the same or different, is a straight or branched chain alkyl radical of 1 to 14 carbon atoms,
or a glucuronide group

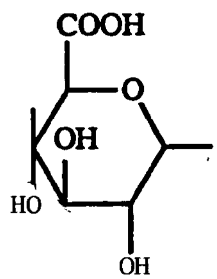

hydrogen atom at position 5 is present in the α- or β- configuration or a mixture of both configurations, and from 0.1 to 10 parts by weight of polyvinylpyrrolidone, adsorbed on a cross-linked polyvinylpyrrolidone in a ratio of 1 part by weight of said mixture to 0.20 to 20 parts by weight of cross-linked polyvinylpyrrolidone, characterised in that it comprises the steps of dissolving the steroid and the polyvinylpyrrolidone in a common solvent in which both the steroid and the polyvinylpyrrolidone are soluble, mixing the solution thereby obtained with a given quantity of the cross-linked polyvinylpyrrolidone so as to permit adsorption of said steroid and said polyvinylpyrrolidone to said cross-linked polyvinylpyrrolidone and removing the solvent.

2. A process according to Claim 1, characterised in that the compound prepared is dehydroepiandrosterone or 16-bromoepiandrosterone , or a hydrate, polymorph, enantiomer or isomer thereof, or a salt therof.

3. A process according to Claim 1 or 2, characterised in that the polyvinylpyrrolidone is present in the adsorbate in an amount of 0.1 - 2 parts by weight relative to 1 part by weight of the steroid.

4. A process according to any one of Claims 1 to 3, characterised in that the formulation prepared contains 1 part by weight of said mixture relative to 0.2 - 10 parts by weight of cross-linked polyvinylpyrrolidone.

5. A process according to any one of Claims 1 to 4, characterised in that the polyvinylpyrrolidone has an average molecular weight in the range 65,000 - 250,000.

6. A process according to any one of Claims 1 to 5, characterised in that the adsorbate is blended with a polymeric or mineral material which disintegrates in the presence of water.

7. A process according to Claim 6, characterised in that the material which disintegrates in the presence of water is selected from natural starch, pregelatinised starch, modified corn starch, sodium starch glycolate, sodium carboxymethylcellulose, carboxymethylcellulose, cellulose, methylcellulose, microcrystalline cellulose, ion-exchange resins, cross-linked polyvinylpyrrolidone, clays, alginic acid or salts thereof, and gums.

8. A process according to Claim 7, characterised in that the solvent used is any pharmaceutically suitable common solvent in which both the steroid and the polyvinylpyrrolidone are soluble.

9. A process according to any one of Claims 1 to 8 , characterised in that the in vitro solubility of the steroid in the absorbate prepared is equal to or in excess of 1.1 times the solubility of the free steroid in micronised form.

10. A process according to any one of Claims 1 to 9, characterised in that the formulation prepared is in the form of a powder, granule, tablet, capsule or suspension.

## Patentansprüche

**Patentansprüche für folgende Vertragsstaaten : AT, BE, CH, LI, DE, DK, FR, GB, IT, LU, NL, SE**

1. Adsorbat-Formulierung mit einer vergrößerten Bioverfügbarkeit, umfassend ein Adsorbat aus einem Gemisch von einem Gewichtsteil einer Verbindung der allgemeinen Formel (I)

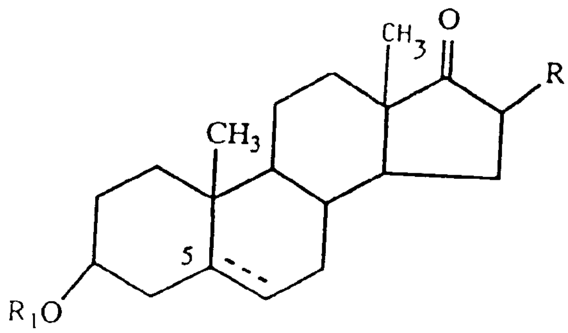

(I)

worin R ein Wasserstoff- oder Bromatom darstellt und $R_1$ steht für ein Wasserstoffatom, eine $SO_2OM$-Gruppe, worin M ein Wasserstoff- oder Natriumatom bedeutet, eine Sulfatid-Gruppe

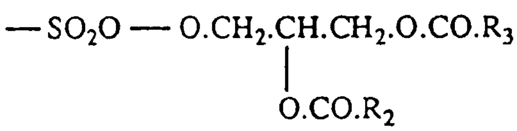

worin $R_2$ und $R_3$, die gleich oder verschieden sein können, jeweils ein geradkettiges oder verzweigtes Alkyl-Radikal mit 1 bis 14 Kohlenstoffatomen darstellen, eine Phosphatid-Gruppe

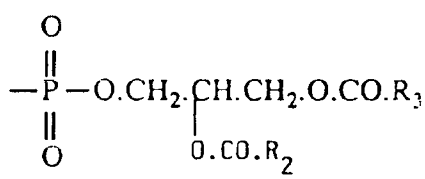

worin $R_2$ und $R_3$, die gleich oder verschieden sein können, jeweils ein geradkettiges oder verzweigtes Alkyl-Radikal mit 1 bis 14 Kohlenstoffatomen darstellen, oder eine Glucuronid-Gruppe

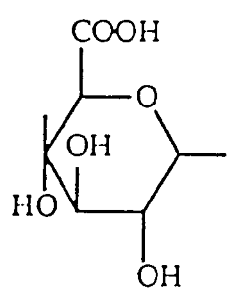

und worin die durchbrochene Linie eine frei wählbare Doppelbindung darstellt und das Wasserstoffatom in Position 5 in der α- oder β-Konfiguration oder als Gemisch aus beiden Konfigurationen vorliegt; und 0,1 bis 10 Gewichtsteilen Polyvinylpyrrolidon, adsorbiert auf vernetztes Polyvinylpyrrolidon in einem Verhältnis von 1 Gewichtsteil des genannten Gemisches zu 0,20 bis 20 Gewichtsteilen vernetztes Polyvinylpyrrolidon.

2. Formulierung nach Anspruch 1, dadurch gekennzeichnet, daß die Verbindung Dehydroepiandrosteron oder 16-Bromepiandrosteron oder ein Hydrat, eine polymorphe Verbindung, ein Enantiomeres oder Isomeres davon oder ein Salz davon ist.

3. Formulierung nach Anspruch 1 oder 2, dadurch gekennzeichnet, daß das Polyvinylpyrrolidon in dem Adsorbat in einer Menge von 0,1 bis 2 Gewichtsteilen, bezogen auf 1 Gewichtsteil Steroid, vorhanden ist.

4. Formulierung nach einem der Ansprüche 1 bis 3, dadurch gekennzeichnet, daß sie 1 Gewichtsteil des genannten Gemisches auf 0,2 bis 10 Gewichtsteile vernetztes Polyvinylpyrrolidon enthält.

5. Formulierung nach einem der Ansprüche 1 bis 4, dadurch gekennzeichnet, daß das Polyvinylpyrrolidon ein durchschnittliches Molekulargewicht im Bereich von 65 000 - 250 000 aufweist.

6. Formulierung nach einem der Ansprüche 1 bis 5, dadurch gekennzeichnet, daß das Adsorbat vermischt ist mit einem polymeren oder mineralischen Material, welches sich in Gegenwart von Wasser auflöst.

7. Formulierung nach Anspruch 6, dadurch gekennzeichnet, daß das Material, das sich in Gegenwart von Wasser auflöst, ausgewählt ist aus natürlicher Starke vorgelierter Stärke, modifizierter Maisstärke, Natriumstarkeglycolat, Natriumcarboxymethylcellulose, Carboxymethylcellulose, Cellulose, Methylcellulose, mikrokristalliner Cellulose, Ionenaustauschharzen, vernetztem Polyvinylpyrrolidon, Tonen, Alginsäure oder Salzen davon und Gummen.

8. Verfahren zur Herstellung einer Formulierung mit vergrößerter Bioverfügbarkeit nach einem der Ansprüche 1 bis 7, dadurch gekennzeichnet, daß es umfaßt die Stufen Auflösen von Steroid und Polyvinylpyrrolidon in einem gemeinsamen Lösungsmittel, in dem sowohl das Steroid als auch das Polyvinylpyrrolidon löslich sind, Vermischen der so erhaltenen Lösung mit einer gegebenen Menge des vernetzten Polyvinylpyrrolidon, so daß eine Adsorption des genannten Steroids und des genannten Polyvinylpyrrolidon an das genannte vernetzte Polyvinylpyrrolidon ermöglicht wird, und Entfernen des Lösungsmittels.

9. Formulierung nach einem der Ansprüche 1 bis 7, dadurch gekennzeichnet, daß die in vitro-Löslichkeit des Steroids in dem Absorbat gleich ist oder dem 1,1fachen der Löslichkeit des freien Steroids in Form einer Größenordnung im µm-Bereich entspricht.

10. Formulierung nach einem der Ansprüche 1 bis 7 oder 9, dadurch gekennzeichnet, daß sie in Form eines Pulvers, Körnchens, einer Tablette, Kapsel oder Suspension vorliegt.

**Patentansprüche für folgende Vertragsstaaten : ES, GR**

1. Verfahren zur Herstellung einer Adsorbat-Formulierung mit verstärkter Bioverfügbarkeit, umfassend ein Adsorbat eines Gemisches aus einem Gewichtsteil einer Verbindung der allgemeinen Formel (I)

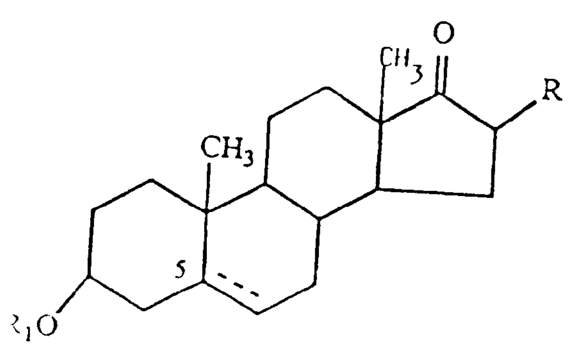

(I)

worin R für ein Wasserstoff- oder Bromatom steht und $R_1$ steht für ein Wasserstoffatom, eine $SO_2OM$-Gruppe, worin M ein Wasserstoff- oder Natriumatom bedeutet, eine Sulfatid-Gruppe

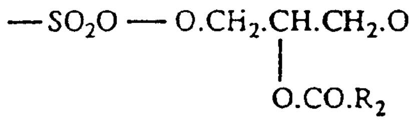

worin $R_2$ und $R_3$, die gleich oder verschieden sein können, jeweils ein geradkettiges oder verzweigtes Alkyl-Radikal mit 1 bis 14 Kohlenstoffatomen darstellen, eine Phosphatid-Gruppe

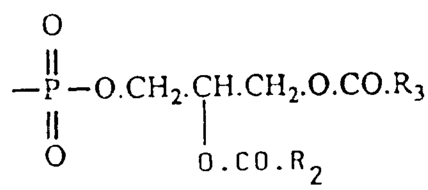

worin jeweils $R_2$ und $R_3$, die gleich oder verschieden sein können, ein geradkettiges oder verzweigtes Alkyl-Radikal mit 1 bis 14 Kohlenstoffatomen darstellen, oder eine Glucuronid-Gruppe

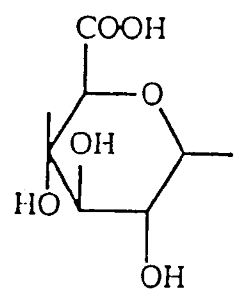

und worin die durchbrochene Linie eine frei wahlbare Doppelbindung darstellt und das Wasserstoffatom in Position 5 in der α- oder β-Konfiguration oder als Gemisch aus beiden Konfigurationen vorliegt; und 0,1 bis 10 Gewichtsteilen Polyvinylpyrrolidon, adsorbiert auf einem vernetzten Polyvinylpyrrolidon in einem Verhältnis von 1 Gewichtsteil des genannten Gemisches zu 0,20 bis 20 Gewichtsteilen vernetztes Polyvinylpyrrolidon, dadurch gekennzeichnet, daß es umfaßt die Stufen Auflösen von Steroid und Polyvinylpyrrolidon in einem gemeinsamen Lösungsmittel, in dem sowohl das Steroid als auch das Polyvinylpyrrolidon löslich sind, Vermischen der so erhaltenen Lösung mit einer gegebenen Menge des vernetzten Polyvinylpyrrolidon, so daß eine Adsorption des genannten Steroids und des genannten Polyvinyl-pyrrolidon an das genannte vernetzte Polyvinylpyrrolidon ermöglicht wird, und Entfernen des Lösungsmittels.

2. Verfahren nach Anspruch 1, dadurch gekennzeichnet, daß die hergestellte Verbindung Dehydroepiandrosteron oder 16-Bromepiandrosteron oder ein Hydrat, eine polymorphe Verbindung, ein Enantiomeres oder Isomeres davon oder ein Salz davon ist.

3. Verfahren nach Anspruch 1 oder 2, dadurch gekennzeichnet, daß das Polyvinylpyrrolidon in dem Adsorbat in einer Menge von 0,1 bis 2 Gewichtsteilen auf 1 Gewichtsteil Steroid vorhanden ist.

4. Verfahren nach einem der Ansprüche 1 bis 3, dadurch gekennzeichnet, daß die hergestellte Formulierung 1 Gewichtsteil des genannten Gemisches auf 0,2 bis 10 Gewichtsteile vernetztes Polyvinylpyrrolidon enthält.

5. Verfahren nach einem der Ansprüche 1 bis 4, dadurch gekennzeichnet, daß das Polyvinylpyrrolidon ein durchschnittliches Molekulargewicht im Bereich von 65 000 - 250 000 aufweist.

6. Verfahren nach einem der Ansprüche 1 bis 5, dadurch gekennzeichnet, daß das Adsorbat vermischt wird mit einem polymeren oder mineralischen Material, das sich in Gegenwart von Wasser auflöst.

7. Verfahren nach Anspruch 6, dadurch gekennzeichnet, daß das Material, das sich in Anwesenheit von Wasser auflöst, ausgewählt ist aus natürlicher Stärke, vorgelierter Stärke, modifizierter Maisstärke, Natriumstärkeglycolat, Natriumcarboxymethylcellulose, Carboxymethylcellulose, Cellulose, Methylcellulose, mikrokristalline Cellulose, Ionenaustauschharzen, vernetztem Polyvinylpyrrolidon, Tonen, Alginsäure oder Salzen davon und Gummen.

8. Verfahren nach Anspruch 7, dadurch gekennzeichnet, daß das verwendete Lösungsmittel irgendein pharmazeutisch geeignetes gemeinsames Lösungsmittel ist, in dem sowohl das Steroid als auch das Polyvinylpyrrolidon löslich sind.

9. Verfahren nach einem der Ansprüche 1 bis 8, dadurch gekennzeichnet, daß die in vitro-Löslichkeit des Steroids in dem hergestellten Absorbat gleich oder dem 1,1fachen der Löslichkeit des freien Steroids in Form einer Größenordnung im µm-Bereich entspricht.

10. Verfahren nach einem der Ansprüche 1 bis 9, dadurch gekennzeichnet, daß die hergestellte Formulierung in Form eines Pulvers, Körnchens, einer Tablette, Kapsel oder Suspension vorliegt.

**Revendications**

**Revendications pour les Etats contractants suivants : AT, BE, CH, LI, DE, DK, FR, GB, IT, LU, NL, SE**

1. Formulation d'adsorbat à biodisponibilité accrue comprenant un adsorbat d'un mélange d'une partie en poids d'un composé de formule générale (I)

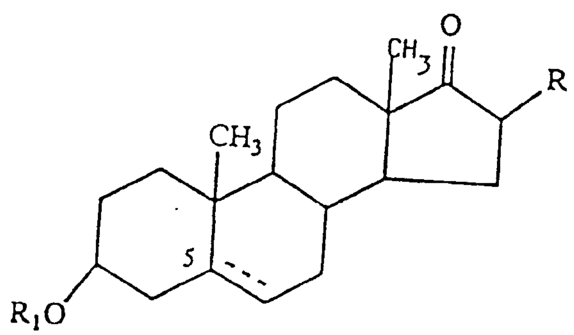

(I)

dans laquelle R est un atome d'hydrogène ou de brome, et $R_1$ est un atome d'hydrogène, un groupe -$SO_2OM$ dans lequel M est un atome d'hydrogène ou de sodium, un groupe sulfatide

$$-SO_2O-O.CH_2.\underset{\displaystyle |\atop \displaystyle O.CO.R_2}{CH}.CH_2.O.CO.R_3$$

dans lequel chacun de $R_2$ et $R_3$, qui peuvent être identiques ou différents, est un radical alkyle à chaîne droite ou ramifiée de 1 à 14 atomes de carbone,
un groupe phosphatide

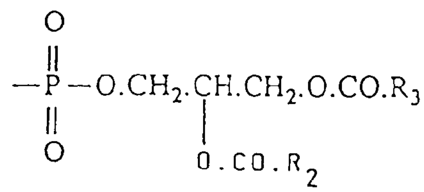

dans lequel chacun de R2 et R3 qui peuvent être identiques ou différents, est un radical alkyle à chaîne droite ou ramifiée de 1 à 14 atomes de carbone,
ou un groupe glucuronide

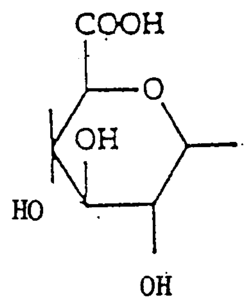

et dans lequel la ligne en pointillés représente une double liaison facultative, et l'atome d'hydrogène en position 5 est présent dans la configuration α ou β ou un mélange des deux configurations, et de 0,1 à 10 parties en poids de polyvinylpyrrolidone, adsorbé sur une polyvinylpyrrolidone réticulée dans un rapport de 1 partie en poids dudit mélange à 0,20 à 20 parties en poids de polyvinylpyrrolidone réticulée.

**2.** Formulation selon la revendication 1, caractérisée en ce que le composé est la déhydroépiandrostérone ou la 16-bromoépiandrostérone, ou un hydrate, un polymorphe, un énantiomère, ou un isomère de ces composés, ou un sel de ces composés.

**3.** Formulation selon la revendication 1 ou 2, caractérisée en ce que la polyvinylpyrrolidone est présente dans l'adsorbat à raison de 0,1 à 2 parties en poids pour 1 partie en poids du stéroïde.

**4.** Formulation selon l'une quelconque des revendications 1 à 3, caractérisée en ce qu'elle contient 1 partie en poids dudit mélange pour 0,2 à 10 parties en poids de polyvinylpyrrolidone réticulée.

**5.** Formulation selon l'une quelconque des revendications 1 à 4, caractérisée en ce que la polyvinylpyrrolidone a un poids moléculaire moyen dans l'intervalle de 65000 à 250000.

**6.** Formulation selon l'une quelconque des revendications 1 à 5, caractérisée en ce que l'adsorbat est mélangé à un matériau polymère ou minéral qui se désintègre en présence d'eau.

**7.** Formulation selon la revendication 6, caractérisée en ce que le matériau qui se désintègre en présence d'eau est choisi parmi l'amidon naturel, l'amidon prégélatinisé, l'amidon de maïs, modifié, le glycolate de sodium d'amidon, la carboxyméthylcellulose de sodium, la carboxyméthylcellulose, la cellulose, la méthylcellulose, la cellulose microcristalline , les résines échangeuses d'ions, la polyvinylpyrrolidone réticulée, les argiles, l'acide alginique ou des sels de cet acide, et des gommes.

**8.** Procédé de préparation d'une formulation à biodisponibilité accrue selon l'une des revendications 1 à 7, caractérisé en ce qu'il comprend les étapes de dissolution du stéroïde et de la polyvinylpyrrolidone dans un solvant commun dans lequel le stéroïde et la polyvinylpyrrolidone sont solubles, le mélange de la solution ainsi obtenue avec une quantité donnée de la polyvinylpyrrolidone réticulée de manière à permettre l'adsorption dudit stéroïde et de la dite polyvinylpyrrolidone sur la dite polyvinylpyrrolidone réticulée et d'élimination du solvant.

**9.** Formulation selon l'une quelconque des revendications 1 à 7, caractérisée en ce que la solubilité in vitro du stéroïde dans l'adsorbat est égale à ou excède de 1,1 fois la solubilité du stéroïde libre sous forme

micronisée.

10. Formulation selon l'une quelconque des revendications 1 à 7 ou 9, caractérisée en ce qu'elle est sous forme de poudre, granule, tablette, capsule ou suspension.

**Revendications pour les Etats contractants suivants : ES, GR**

1. Procédé pour la préparation d'une formulation d'adsorbat à biodisponibilité accrue comprenant un adsorbat d'un mélange d'une partie en poids d'un composé de formule générale (I)

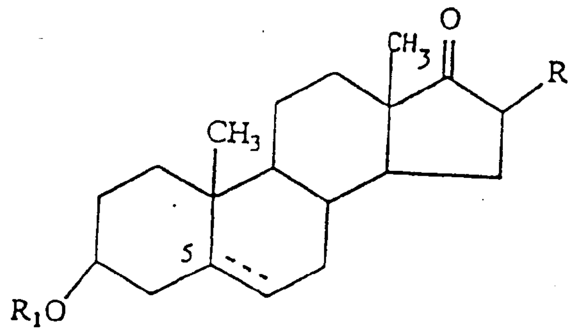

(I)

dans laquelle R est un atome d'hydrogène ou de brome, et $R_1$ est un atome d'hydrogène, un groupe -$SO_2OM$ dans lequel M est un atome d'hydrogène ou de sodium, un groupe sulfatide

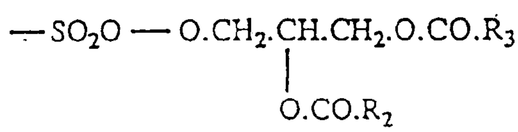

dans lequel chacun de $R_2$ et $R_3$ qui peuvent être identiques ou différents, est un radical alkyle à chaîne droite ou ramifiée de 1 à 14 atomes de carbone,
un groupe phosphatide

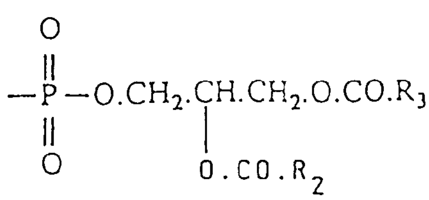

dans lequel chacun de R2 et R3 qui peuvent être identiques ou différents, est un radical alkyle à chaîne droite ou ramifiée de 1 à 14 atomes de carbone, ou un groupe glucuronide

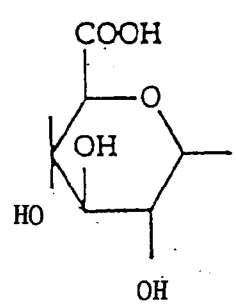

et dans lequel la ligne en pointillés représente une double liaison facultative, et l'atome d'hydrogène en

position 5 est présent dans la configuration α ouβ ou un mélange des deux configurations, et de 0,1 à 10 parties en poids de polyvinylpyrrolidone, adsorbé sur une polyvinylpyrrolidone réticulée dans un rapport de 1 partie en poids dudit mélange à 0,20 à 20 parties en poids de polyvinylpyrrolidone réticulée, caractérisé en ce qu'il comprend les étapes de dissolution du stéroïde et de la polyvinylpyrrolidone dans un solvant commun dans lequel le stéroïde et la polyvinylpyrrolidone sont solubles, le mélange de la solution ainsi obtenue avec une quantité donnée de la polyvinylpyrrolidone réticulée de manière à permettre l'adsorption dudit stéroïde et de ladite polyvinylpyrrolidone sur ladite polyvinylpyrrolidone réticulée et d'élimination du solvant.

**2.** Procédé selon la revendication 1, caractérisé en ce que le composé préparé est la déhydroépiandrostérone ou la 16-bromoépiandrostérone, ou un hydrate, un polymorphe, un énantiomère ou un isomère de ces composés, ou un sel de ces composés.

**3.** Procédé selon la revendication 1 ou 2, caractérisé en ce que la polyvinylpyrrolidone est présente dans l'adsorbat à raison de 0,1 à 2 parties en poids pour 1 partie en poids de stéroïde.

**4.** Procédé selon l'une quelconque des revendications 1 à 3, caractérisé en ce que la formulation préparée contient 1 partie en poids dudit mélange pour 0,2 à 10 parties en poids de polyvinylpyrrolidone réticulée.

**5.** Procédé selon l'une quelconque des revendications 1 à 4, caractérisé en ce que la polyvinylpyrrolidone a un poids moléculaire moyen de l'ordre de 65000 à 250000.

**6.** Procédé selon l'une quelconque des revendications 1 à 5, caractérisé en ce que l'adsorbat est mélange à un matériau polymère ou minéral qui se désintègre en présence d'eau.

**7.** Procédé selon la revendication 6, caractérisé en ce que le matériau qui se désintègre en présence d'eau est choisi parmi l'amidon naturel, l'amidon prégélatinisé, l'amidon de maïs modifié, le glycolate de sodium d'amidon, la carboxyméthylcellulose de sodium, la carboxyméthylcellulose, la cellulose, la méthylcellulose, la cellulose microcristalline, les résines échangeuses d'ions, la polyvinylpyrrolidone réticulée, les argiles, l'acide alginique ou des sels de cet acide, et des gommes.

**8.** Procédé selon la revendication 7, caractérisé en ce que le solvant utilisé est un solvant quelconque pharmaceutiquement approprié dans lequel le stéroïde et la polyvinylpyrrolidone sont solubles.

**9.** Procédé selon l'une quelconque des revendications 1 à 8, caractérisé en ce que la solubilité in vitro du stéroïde dans l'adsorbat préparé est égale à ou excède de 1,1 fois la solubilité du stéroïde libre sous forme micronisée.

**10.** Procédé selon l'une quelconque des revendications 1 à 9, caractérisé en ce que la formulation préparée est sous forme de poudre, granule, tablette, capsule ou suspension.

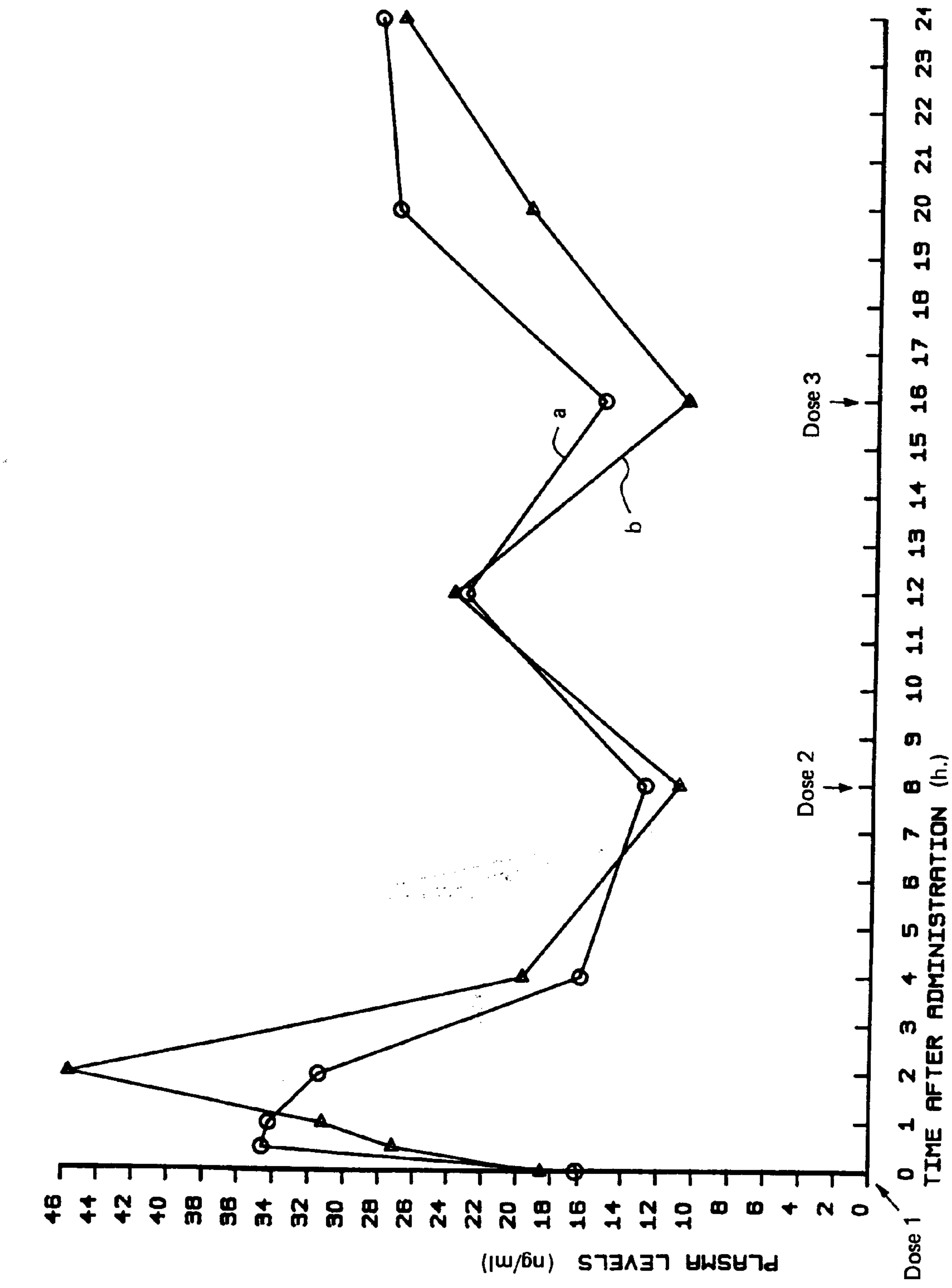
PLASMA LEVELS (ng/ml)
TIME AFTER ADMINISTRATION (h.)
Dose 1
Dose 2
Dose 3
a
b

FIG. 1

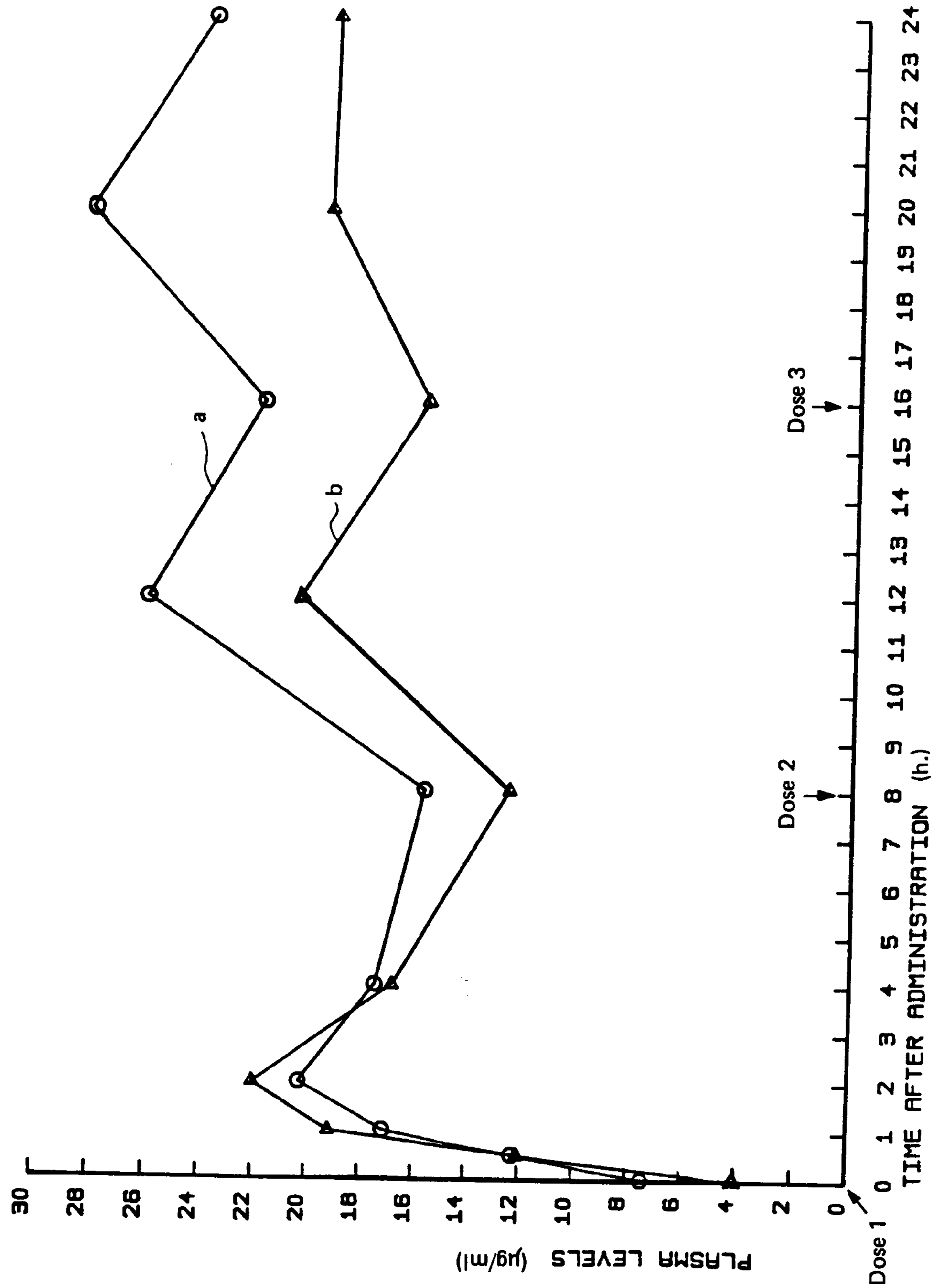
PLASMA LEVELS (µg/ml)
0
2
4
6
8
10
12
14
16
18
20
22
24
26
28
30
TIME AFTER ADMINISTRATION (h.)
0
1
2
3
4
5
6
7
8
9
10
11
12
13
14
15
16
17
18
19
20
21
22
23
24
Dose 1
Dose 2
Dose 3
a
b

FIG. 2